# EUROPEAN PATENT APPLICATION

(11) **EP 4 792 740 A1**
(43) Date of publication of application: **19.08.2026**
(21) Application number: 24890672.9
(22) Date of filing: 12.11.2024
(51) Int. Cl.: A61B 5/026, A61B 3/00

(54) **BLOOD FLOW IMAGE COMPUTING METHOD AND APPARATUS, AND ELECTRONIC DEVICE**

(30) Priority: 16.11.2023 CN 202311533691
(71) Applicant: Intalight Group Co., Ltd., Luoyang, Henan 471003 (CN)
(72) Inventor: WANG, Zhengyu, Luoyang, Henan 471003 (CN); WEI, Xing, Luoyang, Henan 471003 (CN); LI, Bing, Luoyang, Henan 471003 (CN)
(74) Representative: Locas, Davide
(86) International application number: PCT/CN2024/131608
(87) International publication number: WO 2025/103317

(57) **Abstract**

A blood flow image computing method and apparatus, and an electronic device. The method comprises: acquiring a scanning image set, the scanning image set comprising at least two OCT images that are obtained by scanning a target position; and performing Fourier transform for each of the OCT images so as to obtain a complex-amplitude image; and on the basis of at least two preset convolution kernels, performing convolution computing on each convolution kernel and each complex-amplitude image. The complex-amplitude images obtained after Fourier transform undergo convolution computing by means of using the convolution kernels, and then the computing is carried out on the basis of a plurality of complex images corresponding to each convolution kernel. Therefore, the relationship between the amplitudes and phases of adjacent pixels is taken into account, thereby improving the accuracy of computing results.

## Description

### CROSS-REFERENCE TO RELATED APPLICATION

The present invention claims priority to Chinese Patent Application No. 2023115336917, filed with the China National Intellectual Property Administration on November 16, 2023, entitled "BLOOD FLOW IMAGE COMPUTING METHOD AND APPARATUS, AND ELECTRONIC DEVICE", the entire contents of which are incorporated herein by reference.

### TECHNICAL FIELD

The present invention relates to the field of image processing technology, and more particularly, to an OCTA image computing method, apparatus, electronic device, and storage medium for Optical Coherence Tomography (OCT).

### BACKGROUND ART

Optical Coherence Tomography Angiography (OCTA) is an important non-invasive diagnostic technology for fundus diseases that has emerged in recent years.

Currently, an OCTA image computing method is based on continuously acquiring multiple B-scan images at a single scanning position and analyzing the differences between these B-scan images to obtain OCTA information. Existing computing methods mostly employ one-to-one mapping where one output pixel corresponds to a single pixel at the same position in the input images, without considering the possible relationships in amplitude and phase between adjacent pixels, resulting in insufficient accuracy of the analysis results.

### SUMMARY

In view of the foregoing, an object of the present invention is to provide an OCTA image computing method, apparatus, and electronic device.

In a first aspect, embodiments of the present invention provide an OCTA image computing method, the method comprising:
acquiring a scanned image set, wherein the scanned image set comprises at least two OCT images obtained by scanning a target position;
for each of the OCT images, performing Fourier transform on the OCT image to obtain a complex amplitude image;
based on at least two preset convolution kernels, performing convolution calculation on each of the complex amplitude images with each of the convolution kernels to obtain a sub-image set corresponding to each of the convolution kernels;
determining a first image set based on all of the sub-image sets; and
computing images in the first image set to obtain an OCTA image.

In combination with the first aspect, the step of computing the images in the first image set to obtain the OCTA image comprises:
based on a preset pairing rule, pairing images in the sub-image set corresponding to each of the convolution kernels to obtain image pairs;
for each of the image pairs, performing decorrelation calculation on the image pair to obtain a first intermediate result corresponding to the image pair;
determining a first target result corresponding to each of the convolution kernels based on all of the first intermediate results; and
computing based on all of the first target results to obtain the OCTA image.

In combination with the first aspect, the step of for each of the image pairs, performing decorrelation calculation on the image pair comprises:
computing according to the following formula:
   ${D}_{\left(a,a+1, i\right)} = \left|\left|{CK}_{\left(a, i\right)}\right|-\left|{CK}_{\left(a+1, i\right)}\right|\right| / \left(\left|{CK}_{\left(a, i\right)}\right|+\left|{CK}_{\left(a+1, i\right)}\right|+Sigma\right) ,$
wherein D_{(a, a+1, i)} is the first intermediate result obtained by performing decorrelation calculation on an image pair formed by the a-th complex amplitude image and the (a+1)-th complex amplitude image after convolution calculation with the i-th convolution kernel respectively; CK_{(a, i)} is a complex image obtained by convolution calculation of the a-th complex amplitude image with the i-th convolution kernel, CK_{(a+1, i)} is a complex image obtained by convolution calculation of the (a+1)-th complex amplitude image with the i-th convolution kernel, and Sigma is zero or a positive number.

In combination with the first aspect, the step of for each of the image pairs, performing decorrelation calculation on the image pair comprises:
computing according to the following formula:
${D}_{\left(a, a+1, i\right)} = {\left(\left|{CK}_{\left(a, i\right)}\right|-\left|{CK}_{\left(a+1, i\right)}\right|\right)}^{2} / \left({\left|{CK}_{\left(a, i\right)}\right|}^{2}+{\left|{CK}_{\left(a+1, i\right)}\right|}^{2}+Sigma\right) .$

In combination with the first aspect, after the step of pairing images in the sub-image set corresponding to each of the convolution kernels based on the preset pairing rule to obtain image pairs, the method further comprises:
for each of the image pairs, determining whether absolute values of a first complex image and a second complex image in the image pair are both less than a first predetermined threshold;
if yes, determining that the first intermediate result corresponding to the image pair is a preset value.

In combination with the first aspect, the step of determining the first target result corresponding to each of the convolution kernels based on all of the first intermediate results comprises:
determining the first target result corresponding to each of the convolution kernels by taking an average result or a median of all of the first intermediate results corresponding to each of the convolution kernels.

In combination with the first aspect, the step of computing based on all of the first target results to obtain the OCTA image comprises:
acquiring a weight corresponding to each of the convolution kernels; and
computing the OCTA image based on the weights corresponding to the convolution kernels and all of the first target results.

In combination with the first aspect, the step of computing the OCTA image based on the weights corresponding to the convolution kernels and all of the first target results comprises:
summing products of the weights corresponding to individual convolution kernels and the first target results corresponding to the convolution kernels to obtain the OCTA image.

In combination with the first aspect, the step of computing the OCTA image based on the weights corresponding to the convolution kernels and all of the first target results comprises:
computing the OCTA image according to the following formula:
   $Angio = \sum \left({D}_{i}\times {W}_{i}\right) \div \sum {W}_{i} ;$
wherein Angio is the output OCTA image, Dᵢ is the first target result corresponding to the i-th convolution kernel, and Wᵢ is the weight corresponding to the i-th convolution kernel.

In combination with the first aspect, the step of computing based on all of the first target results to obtain the OCTA image comprises:
averaging all of the first target results, and using the obtained average result as the OCTA image.

In combination with the first aspect, after the step of acquiring the scanned image set, the method further comprises:
inputting the scanned image set into a preset model, and outputting the OCTA image.

In combination with the first aspect, a training process of the model is as follows:
acquiring training samples, wherein the training samples comprise a first quantity of images;
computing the first quantity of images according to a preset algorithm to obtain a first OCTA image;
using the first OCTA image as a target result, and initializing convolution kernels and weights;
selecting a second quantity of images from the first quantity of images as a second image set, wherein the second quantity is less than the first quantity;
computing images in the second image set using the OCTA image computing method according to the initialized convolution kernels and weights to obtain a second OCTA image;
computing a residual between the second OCTA image and the target result;
accumulating the residual to obtain a loss function of the model; and
optimizing the model according to the loss function until a preset condition is satisfied to obtain an optimized model, wherein the optimized model comprises optimized convolution kernels and weights corresponding to the optimized convolution kernels.

In combination with the first aspect, the step of computing the first quantity of images according to the preset algorithm to obtain the first OCTA image comprises:
computing the output OCTA image according to the following formula:
   $Angio = \frac{1}{M - 1} \sum \frac{\left|\left|{C}_{i}\right|-\left|{C}_{i + 1}\right|\right|}{\left(\left|{C}_{i}\right|+\left|{C}_{i + 1}\right|+Sigma\right)} ;$
wherein Angio is the output OCTA image, Cᵢ is the complex amplitude image corresponding to the i-th image, Sigma is zero or a positive number, and Sigma is determined according to system noise, and M is the first quantity; and
performing low-pass filtering and denoising processing on the output OCTA image to obtain the first OCTA image.

In combination with the first aspect, the step of initializing the convolution kernels and weights comprises:
assuming the number of convolution kernels is n, setting a center element of the first convolution kernel to 1 and remaining elements to 0; setting all elements at a distance of 1 from the center of the second convolution kernel to 1/L, where L is the number of all non-zero elements, and remaining elements to 0; and so on;
initializing the weights to ${W}_{i} = \frac{1}{n}$, wherein Wᵢ is the weight corresponding to the i-th convolution kernel.

In combination with the first aspect, before the step of performing Fourier transform on the OCT image, the method further comprises:
performing dispersion correction processing on the OCT image to correct dispersion of the image.

In combination with the first aspect, after the step of performing Fourier transform on the OCT image to obtain the complex amplitude image, the method further comprises:
for each of the complex amplitude images, computing an absolute value of a signal corresponding to the complex amplitude image and using the absolute value as a real amplitude image;
for each of the real amplitude images, computing a logarithm of a signal corresponding to the real amplitude image to obtain a first structural image;
selecting one of the first structural images as a reference image, and determining first structural images other than the reference image as images to be corrected;
for each of the images to be corrected, computing a misalignment amount between the reference image and the image to be corrected; and
correcting complex amplitude images corresponding to the images to be corrected based on all of the misalignment amounts to obtain corrected complex amplitude images.

In a second aspect, the present invention provides an OCTA image computing apparatus, the apparatus comprising:
an image acquisition module, configured to acquire a scanned image set, wherein the scanned image set comprises at least two OCT images obtained by scanning a target position;
an image transform module, configured to, for each OCT image, perform Fourier transform on the OCT image to obtain a complex amplitude image;
a convolution calculation module, configured to, based on at least two preset convolution kernels, perform convolution calculation on each of the complex amplitude images with each of the convolution kernels to obtain a sub-image set corresponding to each of the convolution kernels;
a determination module, configured to determine a first image set based on all of the sub-image sets; and
an OCTA image computing module, configured to compute images in the first image set to obtain an OCTA image.

In a third aspect, the present invention provides an electronic device, comprising: a memory and a processor, wherein the memory stores a computer program, and the processor is configured to execute the computer program to implement the method of the above first aspect.

In a fourth aspect, the present invention provides a computer-readable storage medium, wherein a computer program is stored on the computer-readable storage medium, and the computer program, when executed by a processor, implements the method of the first aspect.

In a fifth aspect, the present invention provides a computer program product, comprising a computer program, wherein the computer program, when executed by a processor, implements the method of the first aspect.

The embodiments of the present invention bring the following beneficial effects: the present invention provides an OCTA image computing method, apparatus, and electronic device, the method comprising: acquiring a scanned image set, wherein the scanned image set comprises at least two OCT images obtained by scanning a target position; for each of the OCT images, performing Fourier transform on the OCT image to obtain a complex amplitude image; based on at least two preset convolution kernels, performing convolution calculation on each of the complex amplitude images with each of the convolution kernels to obtain a sub-image set corresponding to each of the convolution kernels; determining a first image set based on all of the sub-image sets; and computing images in the first image set to obtain an OCTA image.

In the present invention, convolution calculation is performed on Fourier-transformed complex amplitude images using convolution kernels, and then calculation is performed on multiple complex images corresponding to each convolution kernel, taking into account the amplitude and phase relationships between adjacent pixels to improve the accuracy of calculation results, and then the OCTA image is computed based on all of the first target results to fully optimize the signal-to-noise ratio of the OCTA image, thereby improving the accuracy of the OCTA image.

Other features and advantages of the present invention will be set forth in the description that follows, and in part will become apparent from the description, or may be learned by practice of the present invention. The objects and other advantages of the present invention will be realized and obtained by means of the structures particularly pointed out in the description, claims, and drawings hereof.

In order to make the above objects, features, and advantages of the present invention more clearly comprehensible, preferred embodiments are described in detail below with reference to the drawings.

### BRIEF DESCRIPTION OF DRAWINGS

In order to more clearly illustrate the technical solutions of specific embodiments of the present invention or the prior art, a brief introduction to the drawings required for describing the specific embodiments or the prior art is provided below. Obviously, the drawings in the following description are merely some embodiments of the present invention. For those skilled in the art, other drawings may be obtained based on these drawings without creative effort.
FIG. 1 is a flowchart of an OCTA image computing method provided by an embodiment of the present invention;
FIG. 2 is a schematic structural diagram of an OCTA image computing apparatus provided by an embodiment of the present invention;
FIG. 3 is a schematic structural diagram of an electronic device provided by an embodiment of the present invention;
FIG. 4 is a first OCTA image obtained by computing collected B-scan images according to a preset algorithm in the related art; and
FIG. 5 is a second OCTA image obtained by performing convolution calculation on collected B-scan images using the method provided by an embodiment of the present invention.

### Reference Numerals:

10 - Image acquisition module, 20 - Image transform module, 30 - Convolution calculation module, 40 - Determination module, 50 - OCTA image computing module;
41 - Processor, 42 - Bus, 43 - Communication interface, 44 - Memory.

### DETAILED DESCRIPTION OF EMBODIMENTS

In order to make the objects, technical solutions, and advantages of the embodiments of the present invention clearer, the technical solutions of the present invention will be clearly and completely described below in conjunction with the drawings. Obviously, the described embodiments are merely a part of the embodiments of the present invention, rather than all of the embodiments. Based on the embodiments of the present invention, all other embodiments obtained by those skilled in the art without creative effort shall fall within the protection scope of the present invention.

In order to facilitate understanding of the present embodiment, technical terms involved in the present invention are briefly introduced below.

OCT (Optical Coherence Tomography) technology has been widely applied in the diagnosis of fundus diseases and is of great significance for the detection and treatment of ophthalmic diseases. Using the coherence of light to scan and image the fundus, each scan in the depth direction at the same lateral position is called an A-scan, and multiple adjacent continuous scans combined together are called a B-scan. A B-scan image is also commonly referred to as an OCT sectional image. The OCT image is an image obtained by scanning a target position using an OCT device, and the OCT image may be an A-scan image or a B-scan image. Compared with traditional methods, this method not only does not require OCTA contrast agents, but can also clearly image large and medium vessels and capillaries in multiple physiological structure layers within the retina. It can be used to diagnose various fundus diseases such as macular degeneration, neovascularization, diabetic retinopathy, vascular occlusion, and central serous chorioretinopathy.

After introducing the technical terms involved in the present invention, the application scenario and design concept of the embodiments of the present invention are briefly introduced below.

Existing analysis methods mostly employ one-to-one mapping where one output pixel corresponds to a single pixel at the same position in the input images, without considering the possible relationships in amplitude and phase between adjacent pixels statistically.

Based on this, the embodiments of the present invention provide an OCTA image computing method, apparatus, and electronic device. After acquiring a scanned image set, convolution calculation is performed on each B-scan image in the scanned image set using convolution kernels to introduce the interrelationships in phase and amplitude between a pixel and surrounding different pixels, and then the final OCTA image is calculated. In this way, the signal-to-noise ratio and accuracy of the OCTA image can be improved to obtain accurate OCTA calculation results. The OCTA computing method provided in this embodiment is applied to a processor 41 in an electronic device. The electronic device further comprises a memory 44, wherein the memory 44 stores a computer program, and the processor 41 executes the computer program to implement the method provided by the embodiments of the present invention.

### Embodiment 1

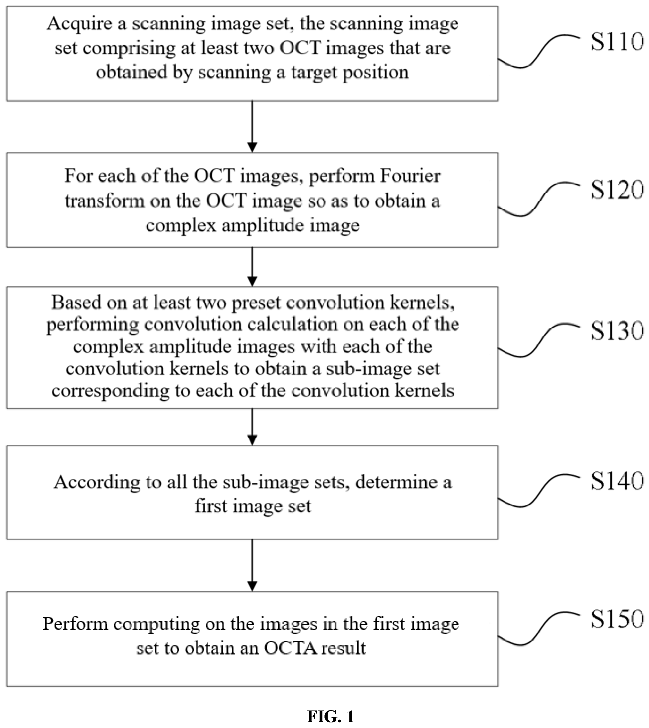

This embodiment provides an OCTA image computing method. As shown in FIG. 1, the method comprises:
S110, acquiring a scanned image set, wherein the scanned image set comprises at least two OCT images obtained by scanning a target position;
S120, for each OCT image, performing Fourier transform on the OCT image to obtain a complex amplitude image;
S130, based on at least two preset convolution kernels, performing convolution calculation on each of the complex amplitude images with each of the convolution kernels to obtain a sub-image set corresponding to each of the convolution kernels;
S140, determining a first image set based on all of the sub-image sets; and
S150, computing images in the first image set to obtain an OCTA image.

In this embodiment, step S110 can comprise: obtaining at least two B-scan images by scanning a target position using an OCT device or an OCTA device. As an implementable manner, the scanning position is a set position, the depth direction is defined as the z-axis of the coordinate system of the eye to be detected, and the x-axis and y-axis are planned in a plane perpendicular to the z-axis. The x-axis in the plane coordinate system is used as the fast scan direction, and the y-axis is used as the slow scan direction, and at least two B-scan images are continuously acquired in the same slow scan direction. In this embodiment, the number M of B-scan images can be 4.

In combination with the above example, step S120 can comprise: for each OCT image, performing Fourier transform on the OCT image to obtain a complex amplitude image corresponding to each OCT image, that is, obtain M complex amplitude images Cᵢ (i=1, ..., M).

In step S130, the number of preset convolution kernels is N, denoted as Kᵢ (i=1, ..., N), and these N convolution kernels are respectively used to convolution calculation with the M complex amplitude images obtained in step S120 to obtain a sub-image set corresponding to each convolution kernel. Through S130, the amplitude and phase relationships between adjacent pixels are taken into account to improve the accuracy of calculation results.

For example, the preset N convolution kernels Kⱼ (j=1, ..., N), N≥2, where N=3 is taken as an example here; then each of the above 4 complex amplitude images Cᵢ obtained by the Fourier transform is subjected to convolution calculation with these N convolution kernels to obtain 4×3 complex images CK_{(i, j)}=Cᵢ⊗Kⱼ; where i=1, 2, 3, 4; j=1, 2, 3; ⊗ is the convolution operation.

In step S130, each convolution kernel corresponds to one sub-image set, and the sub-image set comprises M complex images, with a total of N sub-image sets.

In step S140, the N sub-image sets can be combined to form the first image set.

In step S150, calculation is performed on the images in the first image set obtained after convolution calculation processing to obtain the final OCTA image.

Compared with the prior art, this embodiment performs convolution on Fourier-transformed complex amplitude images using convolution kernels, thereby introducing the correlation relationships in phase and amplitude between a pixel and surrounding different pixels, and then performs computing between different images on the convolved results, thereby improving the signal-to-noise ratio and accuracy of the OCTA image.

Optionally, the step S150 of computing images in the first image set to obtain an OCTA image can specifically include:
S151: based on a preset pairing rule, pairing images in the sub-image set corresponding to each convolution kernel to obtain image pairs;
S152: for each image pair, performing decorrelation calculation on the image pair to obtain a first intermediate result corresponding to the image pair;
S153: determining a first target result corresponding to each convolution kernel according to all the first intermediate results; and
S154: performing computing according to all the first target results to obtain an OCTA image.

In this embodiment, the decorrelation calculation is first performed on each sub-image set through steps S151-S152 to obtain the first intermediate result corresponding to each image pair in the sub-image set. Then, in step S153, the first target result corresponding to each convolution kernel is determined by combining all the first intermediate results. Finally, in step S154, the OCTA image is obtained by computing by combining all the first target results.

Taking i=3 as an example, the images in the sub-image set corresponding to convolution kernel K₃ are derived from: M complex amplitude images subjected to convolution calculation with the convolution kernel K₃. That is, the number of complex images corresponding to the convolution kernel K₃ is M. Two adjacent complex images are paired and subjected to decorrelation calculation to obtain the first intermediate result between the two complex images. Herein, "adjacent" means adjacent in acquisition time; and performing decorrelation on two complex images essentially means performing decorrelation calculation on corresponding pixel points on the two complex images.

Considering that the time span between non-adjacent B-scan images is relatively large and eye movement introduces more noise, it is preferable to perform decorrelation calculation between adjacent B-scan images.

For the same convolution kernel K₁, decorrelation calculation is performed between CK (complex images) given by different B-scan images (i.e., between CK₁ and CK₂, CK₂ and CK₃, CK₃ and CK₄) to obtain a corresponding decorrelation result, namely the first intermediate result.

Specifically, as an implementable manner, the step S152 of performing decorrelation calculation on the image pair to obtain the first intermediate result corresponding to the image pair can be calculated according to the following formula:
${D}_{\left(a, a+1, i\right)} = \left|\left|{CK}_{\left(a, i\right)}\right|-\left|{CK}_{\left(a+1, i\right)}\right|\right| / \left(\left|{CK}_{\left(a, i\right)}\right|+\left|{CK}_{\left(a+1, i\right)}\right|+Sigma\right) .$

As another implementable manner, the step S152 of performing decorrelation calculation on the image pair to obtain the first intermediate result corresponding to the image pair is calculated according to the following formula:
${D}_{\left(a, a+1, i\right)} = {\left(\left|{CK}_{\left(a, i\right)}\right|-\left|{CK}_{\left(a+1, i\right)}\right|\right)}^{2} / \left({\left|{CK}_{\left(a, i\right)}\right|}^{2}+{\left|{CK}_{\left(a+1, i\right)}\right|}^{2}+Sigma\right) .$

D₍ₐ, _{a+1, i)} is the first intermediate result obtained by performing decorrelation calculation on an image pair formed by the a-th complex amplitude image and the (a+1)-th complex amplitude image after convolution calculation with the i-th convolution kernel respectively; i is the i-th convolution kernel; CK_{(a, i)} is the complex image obtained by convolution calculation of the a-th complex amplitude image with the i-th convolution kernel; CK_{(a+1, i)} is the complex image obtained by convolution calculation of the (a+1)-th complex amplitude image with the i-th convolution kernel; and Sigma is zero or a positive number, selected according to system noise. When the OCT image signal intensity is very low, the CK value is very low and D tends to 0.

As another implementable manner, after the step S151 of obtaining image pairs, the method further includes:
S1510: for each image pair, determining whether the absolute values of the first complex image and the second complex image in the image pair are both less than a first predetermined threshold; and
if yes, executing step S1511; and
S1511: determining that the first intermediate result corresponding to the image pair is a preset value.

In this embodiment, the preset value is 0.

All the above three manners are implementable; and the third manner may also be implemented in combination with the first or second manner simultaneously, which is not limited herein.

Subsequently, optionally, the step S153 of determining the first target result corresponding to each convolution kernel according to all the first intermediate results can adopt a manner of calculating an average result or taking a median of all the first intermediate results corresponding to each convolution kernel to determine the first target result corresponding to each convolution kernel. It should be noted that the specific calculation manner of the first target result is not limited herein.

With reference to the above example, decorrelation calculation is performed between C₁ and C₂ to obtain D_{(1, 2)}, and D_{(2, 3)} and D_{(3, 4)} are calculated in the same manner.

Then, the first target result is calculated: D = (D_{(1, 2)} + D_{(2, 3)} + D_{(3, 4)}) ÷ 3.

To give another example as follows: when the number of acquired B-scan images in the scanned image set is 2 and the number of convolution kernel is 1, the number of corresponding image pair is 1. In this case, the first intermediate result obtained by calculation is the first target result.

Optionally, the step S154 of computing according to all the first target results to obtain an OCTA image can specifically include:
S1541: acquiring a weight corresponding to each convolution kernel;
S1542: computing to obtain the OCTA image according to the weight corresponding to each convolution kernel and all the first target results.

In step S1541, each convolution kernel corresponds to one weight. In this embodiment, the values of the weight Wᵢ and the convolution kernel Kᵢ are preset based on experience.

For step S1542, in a possible implementation manner, weighted summation can be performed on all the first target results based on the weight corresponding to each convolution kernel to obtain the final OCTA image. Based on this, the OCTA image can be obtained by summing products of the weights corresponding to various convolution kernels and the first target results corresponding to the convolution kernels. A preset weight Wᵢ (i=1, ..., N) corresponding to each convolution kernel is acquired; and the OCTA image is finally obtained by summing products of the weights corresponding to individual convolution kernels and the first target results corresponding to the convolution kernels. In this way, the signal-to-noise ratio of the OCTA image can be fully optimized, thereby improving the accuracy of the OCTA image.

In another possible implementation manner, a weighted average of all the first target results can be taken as the final OCTA image. Based on this, the OCTA image may be calculated according to the following formula:
$Angio = \sum \left({D}_{i}\times {W}_{i}\right) \div \sum {W}_{i} ,$
wherein Angio is the output OCTA image, Dᵢ is the first target result corresponding to the i-th convolution kernel, and Wᵢ is the weight corresponding to the i-th convolution kernel.

Optionally, the step S154 of computing according to all the first target results to obtain an OCTA image can further specifically include:
calculating an average for all the first target results, and taking the obtained average result as the final OCTA image.

### Embodiment 2

In the OCTA image computing method provided in this embodiment, the values of the weight Wᵢ and the convolution kernel Kᵢ are obtained by learning according to a preset model.

Specifically, after step S110, the scanned image set obtained in step S110 can be input into the preset model to output the OCTA image.

In this embodiment, the training process of the model can be as follows, including steps S210~S280.

S210: acquiring training samples, where the training samples include a first number of images.

In this embodiment, OCT data with a relatively large number of repetitions can be collected, for example, 16 repetitions at each scanning position, i.e., M=16 B-scan images are acquired.

S220: computing the first number of images according to a preset algorithm to obtain a first OCTA image.

The preset algorithm refers to an OCTA algorithm without convolution calculation in the related art, and the OCTA image calculated according to the preset algorithm is taken as the first OCTA image Angio_{GT} (ground truth, abbreviated as GT).

In this embodiment, the calculation formula of the preset algorithm can be as follows:
$Angio = \frac{1}{M - 1} \sum \frac{\left|\left|{C}_{i}\right|-\left|{C}_{i + 1}\right|\right|}{\left(\left|{C}_{i}\right|+\left|{C}_{i + 1}\right|+Sigma\right)} ,$
wherein Angio is the output OCTA image, Cᵢ is the complex amplitude image corresponding to the i-th image, Sigma is zero or a positive number, Sigma is determined according to system noise, and M is the first quantity.

Since multiple repeated acquisitions are performed at each scanning position, the target image usually has a very high signal-to-noise ratio. Data with poor signal-to-noise ratio due to eye movement can be manually discarded, and not used as training data.

Furthermore, low-pass filtering and denoising processing can be performed on the OCTA image output by the preset algorithm to obtain the first OCTA image, so as to further improve the signal-to-noise ratio of the first OCTA image.

S230: taking the first OCTA image as a target result, and initializing convolution kernels and weights.

The manner of initializing convolution kernels can be: assuming the number of convolution kernels is n, setting the central element of the first convolution kernel to 1 and the remaining elements to 0; setting all elements of the second convolution kernel at a distance of 1 from the center to 1/L, where L is the number of all non-zero elements, and the remaining elements to 0; and so on.

The weights are initialized to ${W}_{i} = \frac{1}{n}$, where Wᵢ is the weight corresponding to the i-th convolution kernel.

S240: selecting a second quantity of images from the first quantity of images as a second image set, wherein the second quantity is less than the first quantity.

S250: adopting the OCTA image computing method to compute images in the second image set according to the initialized convolution kernels and weighting to obtain a second OCTA image.

The calculation method can be: extracting all adjacent two B-scan images from the same set of data, and computing by using the convolution-based OCTA image algorithm proposed in steps S120-S150 in Embodiment 1 to obtain the second OCTA image.

S260: calculating a residual between the second OCTA image and the target result.

S270: accumulating the residual to obtain a loss function of the model.

The calculation formula of accumulating residual can be:
$Loss = \sum \left|angio-{angio}_{_ GT}\right| ,$
wherein angio is the second OCTA image calculated by using the convolution-based angio algorithm proposed in steps S110-S150, and angio__{GT} is the target image.

S280: optimizing the model according to the loss function until a preset condition is satisfied to obtain an optimized model, wherein the optimized model includes optimized convolution kernels and weights corresponding to the optimized convolution kernels.

Optionally, a gradient descent method can be used to minimize the loss function, so as to optimize and obtain N convolution kernels Kᵢ and weights Wᵢ, which are stored in a memory.

The model used for optimization can be a machine learning model, and the optimized convolution kernels can be used as preset convolution kernels in the OCTA image computing method next time. That is, in actual application of the OCTA image computing method provided in the present invention, the convolution kernels in step S130 may be set based on experience, or may be obtained by training the model. If obtained by model training, the processor acquires the N convolution kernels Kᵢ and weight Wᵢ data in the memory, and then executes steps S120-S150 according to the N convolution kernels Kᵢ and weights Wᵢ to compute the OCTA image.

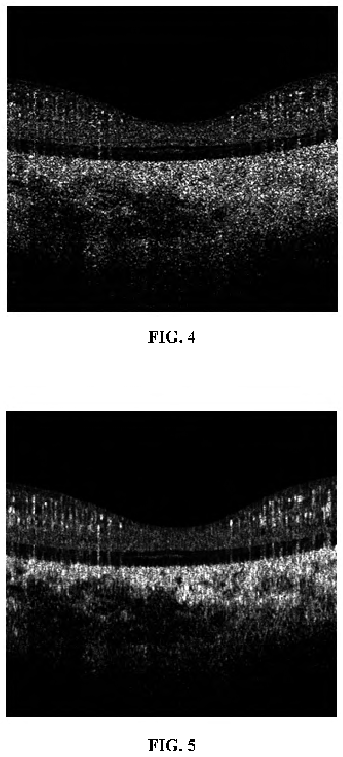

Based on the above method, FIG. 4 exemplarily shows a first OCTA image obtained by computing at a certain scanning position according to a preset algorithm (without convolution calculation). FIG. 5 shows a second OCTA image obtained by performing convolution calculation on the collected images by using the method provided in the embodiment of the present invention at the same acquisition position. It can be seen that the method provided in the embodiment of the present invention helps to improve the signal-to-noise ratio.

Optionally, before the step S120 of performing Fourier transform on the OCT image, the method can further include:
performing dispersion correction processing on the OCT image to correct dispersion of the image.

The dispersion correction may include a third-order polynomial correction method. The third-order polynomial correction method is a manner of performing correction using a phase fitted by a third-order polynomial. The second-order and third-order parameters used in the correction are usually obtained by offline calibration of the system. Since the first-order dispersion correction corresponds to a simple translation for the Fourier-transformed image, it is generally not performed. It should be noted that the specific manner of dispersion correction is not limited in this embodiment.

Optionally, after the step S120 of performing Fourier transform on the OCT image to obtain a complex amplitude image, the method can further include:
S121: for each complex amplitude image, calculating an absolute value of a signal corresponding to the complex amplitude image and taking it as a real amplitude image;
S122: for each real amplitude image, calculating a logarithm of a signal corresponding to the real amplitude image to obtain a first structural image;
S123: selecting one first structural image as a reference image, and determining first structural images other than the reference image as images to be corrected;
S124: for each image to be corrected, calculating a misalignment amount between the reference image and the image to be corrected; and
S125: correcting the complex amplitude image corresponding to the image to be corrected according to all the misalignment amounts to obtain a corrected complex amplitude image.

In this embodiment, the absolute value Aᵢ = |Cᵢ| of the signal corresponding to the complex amplitude image Cᵢ is obtained, and then the logarithm is taken to obtain the first structural image; then misalignment calibration is performed on the first structural image, and the complex amplitude image corresponding to the image to be corrected is corrected according to the calculated misalignment amount.

The calculation process of the first structural image can be expressed as: Sᵢ = 20×log₁₀(Aᵢ) (i=1, ..., 4), wherein Si represents the i-th OCT structural image corresponding to the i-th complex amplitude image.

In actual application, the first OCT structural image corresponding to the complex amplitude image obtained by Fourier transform of the first collected image is usually selected as the reference image.

Then, steps S130-S150 are performed according to the corrected complex amplitude image, so as to further improve the accuracy of data processing.

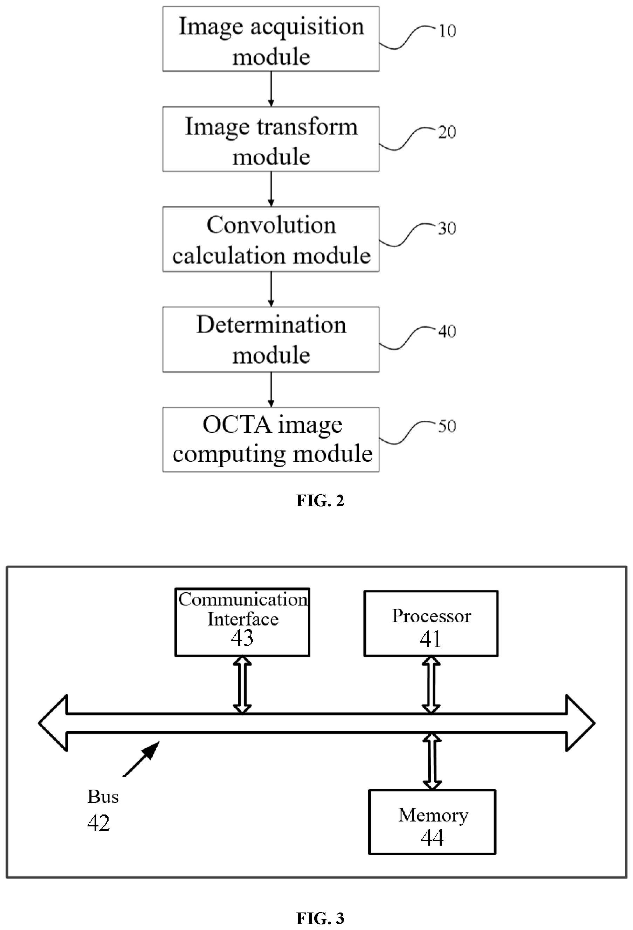

In a second aspect, the present invention provides an OCTA image computing apparatus. As shown in FIG. 2, the apparatus includes: an image acquisition module 10, an image transform module 20, a convolution calculation module 30, a determination module 40, and an OCTA image computing module 50.

The image acquisition module 10 is configured to acquire a scanned image set, where the scanned image set includes at least two OCT images obtained by scanning a target position.

The image transform module 20 is configured to, for each OCT image, perform Fourier transform on the OCT image to obtain a complex amplitude image.

The convolution calculation module 30 is configured to, based on at least two preset convolution kernels, perform convolution calculation on each convolution kernel and each complex amplitude image to obtain a sub-image set corresponding to each convolution kernel.

The determination module 40 is configured to determine a first image set according to all the sub-image sets.

The OCTA image computing module 50 is configured to compute images in the first image set to obtain an OCTA image.

The present invention further provides an electronic device. As shown in FIG. 3, the electronic device includes a memory 44 and a processor 41, the memory 44 stores a computer program, and the processor 41 executes the computer program to implement the above method. As shown in FIG. 3, the electronic device further includes a bus 42 and a communication interface 43, wherein the processor 41, the communication interface 43 and the memory 44 are connected through the bus 42. The memory 44 may include a high-speed random access memory (RAM), and may also include a non-volatile memory, such as at least one magnetic disk memory. The communication connection between the network element of the system and at least one other network element is realized through at least one communication interface 43 (which may be wired or wireless), and the Internet, a wide area network, a local network, a metropolitan area network, etc. may be used. The bus 42 may be an Industry Standard Architecture (ISA) bus, a Peripheral Component Interconnect (PCI) bus, an Extended Industry Standard Architecture (EISA) bus, or the like, and may also be an Advanced Microcontroller Bus Architecture (AMBA) bus, where AMBA defines three types of buses including an Advanced Peripheral Bus (APB) bus, an Advanced High-performance Bus (AHB) bus and an Advanced eXtensible Interface (AXI) bus. The bus 42 may be divided into an address bus, a data bus, a control bus, etc. For ease of representation, only one bidirectional arrow is used in FIG. 4, but it does not mean that there is only one bus or one type of bus.

The processor 41 may be an integrated circuit chip with a signal processing capability. In the implementation process, each step of the above method may be completed by an integrated logic circuit of hardware in the processor 41 or an instruction in a form of software. The processor 41 may be a general-purpose processor including a central processing unit (CPU), a network processor (NP), etc.; and may also be a digital signal processor (DSP), an application specific integrated circuit (ASIC), a field-programmable gate array (FPGA) or other programmable logic devices, discrete gate or transistor logic devices, and discrete hardware components. The general-purpose processor may be a microprocessor or the processor may also be any conventional processor or the like. The steps of the method disclosed in the embodiments of the present invention may be directly embodied as being completed and executed by a hardware decoding processor, or being completed and executed by a combination of hardware and software modules in the decoding processor. The software module may be located in a mature storage medium in the art such as a random access memory, a flash memory, a read-only memory, a programmable read-only memory, an electrically erasable programmable memory, or a register. The storage medium is located in the memory 44, and the processor 41 reads information in the memory 44 and completes the method shown in FIG. 1 in combination with hardware thereof.

The present invention further provides a computer-readable storage medium, where a computer program is stored in the computer-readable storage medium, and the processor 41 executes the computer program to implement the above method. For specific implementation, reference may be made to the method embodiments, and details are not described herein again.

The present invention further provides a computer program product including a computer program, wherein the computer program is executed by a processor to implement the above method.

It can be clearly understood by those skilled in the art that, for convenience and brevity of description, the specific working processes of the system and apparatus described above may refer to the corresponding processes in the foregoing method embodiments, and are not described herein again.

In addition, in the description of the embodiments of the present invention, unless otherwise explicitly specified and defined, the terms "installation", "connection" and "linking" should be understood broadly. For example, the connection may be a fixed connection, a detachable connection, or an integral connection; may be a mechanical connection or an electrical connection; may be a direct connection or an indirect connection through an intermediate medium; and may be internal communication between two elements. For those skilled in the art, the specific meanings of the above terms in the present invention may be understood in specific cases.

If the function is implemented in the form of a software functional unit and is sold or used as an independent product, the function may be stored in a computer-readable storage medium. Based on such understanding, the technical solution of the present invention essentially, or the part contributing to the existing technology, or a part of the technical solution may be embodied in the form of a software product. The computer software product is stored in a storage medium, and includes several instructions for enabling a computer device (which may be a personal computer, a server, or a network device) to execute all or part of the steps of the method described in the embodiments of the present invention. The foregoing storage medium includes: a U disk, a mobile hard disk, a read-only memory (ROM), a random access memory (RAM), a magnetic disk, an optical disk, and other media that can store program codes.

In the description of the present invention, it should be noted that the terms "center", "upper", "lower", "left", "right", "vertical", "horizontal", "inner", "outer" and the like indicate orientations or positional relationships based on those shown in the drawings, and are only for convenience of description of the present invention and simplification of description, but do not indicate or imply that the apparatus or element referred to must be in a specific orientation, be constructed and operated in a specific orientation, and thus should not be construed as limitations to the present invention. Furthermore, the terms "first", "second", and "third" are used for descriptive purposes only and are not to be construed as indicating or implying relative importance.

Finally, it should be noted that the above embodiments are merely specific embodiments of the present invention, which are used to illustrate the technical solutions of the present invention rather than limit it. The protection scope of the present invention is not limited thereto. Although the present invention is described in detail with reference to the foregoing embodiments, those skilled in the art should understand that: any person skilled in the art can still modify or easily conceive of changes to the technical solutions described in the foregoing embodiments or equivalently replace some technical features therein within the technical scope disclosed in the present invention; and such modifications, changes or replacements do not make the essence of the corresponding technical solutions depart from the spirit and scope of the technical solutions of the embodiments of the present invention, and shall fall within the protection scope of the present invention. Therefore, the protection scope of the present invention shall be subject to the protection scope of the claims.

### INDUSTRIAL APPLICABILITY

By applying the technical solution of the present invention, the amplitude and phase relationships between adjacent pixels are taken into account, the accuracy of calculation results is improved, the signal-to-noise ratio of the OCTA image is fully optimized, and thus the accuracy of the OCTA image is improved.

## Claims

1. An OCTA image computing method, **characterized in that** the method comprises:
acquiring a scanned image set, wherein the scanned image set comprises at least two OCT images obtained by scanning a target position;
for each of the OCT images, performing Fourier transform on the OCT image to obtain a complex amplitude image;
based on at least two preset convolution kernels, performing convolution calculation on each of the complex amplitude images with each of the convolution kernels to obtain a sub-image set corresponding to each of the convolution kernels;
determining a first image set based on all of the sub-image sets; and
computing images in the first image set to obtain an OCTA image.

2. The method according to claim 1, wherein the step of computing images in the first image set to obtain an OCTA image comprises:
based on a preset pairing rule, pairing images in the sub-image set corresponding to each of the convolution kernels to obtain image pairs;
for each of the image pairs, performing decorrelation calculation on the image pair to obtain a first intermediate result corresponding to the image pair;
determining a first target result corresponding to each of the convolution kernels based on all of the first intermediate results; and
computing based on all of the first target results to obtain the OCTA image.

3. The method according to claim 2, wherein the step of for each of the image pairs, performing decorrelation calculation on the image pair comprises:
computing according to a following formula: ${D}_{\left(a, a+1, i\right)} = \left|\left|{CK}_{\left(a, i\right)}\right|-\left|{CK}_{\left(a+1, i\right)}\right|\right| / \left(\left|{CK}_{\left(a, i\right)}\right|+\left|{CK}_{\left(a+1, i\right)}\right|+Sigma\right) ,$
wherein D_{(a, a+1, i)} is the first intermediate result obtained by performing the decorrelation calculation on the image pair formed by a-th complex amplitude image and (a+1)-th complex amplitude image after convolution calculation with i-th convolution kernel respectively; CK_{(a, i)} is a complex image obtained by convolution calculation of the a-th complex amplitude image with the i-th convolution kernel; and CK_{(a+1, i)} is a complex image obtained by convolution calculation of the (a+1)-th complex amplitude image with the i-th convolution kernel, and Sigma is zero or a positive number.

4. The method according to claim 2, wherein the step of for each of the image pairs, performing decorrelation calculation on the image pair comprises:
computing according to a following formula: ${D}_{\left(a, a+1, i\right)} = {\left(\left|{CK}_{\left(a, i\right)}\right|-\left|{CK}_{\left(a+1, i\right)}\right|\right)}^{2} / \left({\left|{CK}_{\left(a, i\right)}\right|}^{2}+{\left|{CK}_{\left(a+1, i\right)}\right|}^{2}+Sigma\right) .$

5. The method according to claim 2, wherein after the step of based on a preset pairing rule, pairing images in the sub-image set corresponding to each of the convolution kernels to obtain image pairs, the method further comprises:
for each of the image pairs, determining whether absolute values of a first complex image and a second complex image in the image pair are both less than a first predetermined threshold; and
in response to the absolute values of the first complex image and the second complex image in the image pair being both less than the first predetermined threshold, determining that the first intermediate result corresponding to the image pair is a preset value.

6. The method according to any one of claims 2-5, wherein the step of determining a first target result corresponding to each of the convolution kernels based on all of the first intermediate results comprises:
determining the first target result corresponding to each of the convolution kernels by taking an average result or a median of all of the first intermediate results corresponding to each of the convolution kernel.

7. The method according to any one of claims 2-6, wherein the step of computing based on all of the first target results to obtain the OCTA image comprises:
acquiring a weight corresponding to each of the convolution kernels; and
computing the OCTA image based on the weights corresponding to the convolution kernels and all of the first target results.

8. The method according to claim 7, wherein the step of computing the OCTA image based on the weights corresponding to the convolution kernels and all of the first target results comprises:
summing products of the weights corresponding to individual convolution kernels and the first target results corresponding to the convolution kernels to obtain the OCTA image.

9. The method according to claim 7, wherein the step of computing the OCTA image based on the weights corresponding to the convolution kernels and all of the first target results comprises:
computing the OCTA image according to a following formula: $Angio = \sum \left({D}_{i}\times {W}_{i}\right) \div \sum {W}_{i} ,$
wherein Angio is an output OCTA image, Dᵢ is the first target result corresponding to the i-th convolution kernel, and Wᵢ is the weight corresponding to the i-th convolution kernel.

10. The method according to any one of claims 2-6, wherein the step of computing based on all of the first target results to obtain the OCTA image comprises:
averaging all of the first target results, and using the average result as the OCTA image.

11. The method according to claim 7, wherein after the step of acquiring the scanned image set, the method further comprises:
inputting the scanned image set into a preset model, and outputting the OCTA image.

12. The method according to claim 11, wherein a training process of the model comprises:
acquiring training samples, wherein the training samples comprise a first quantity of images;
computing the first quantity of images according to a preset algorithm to obtain a first OCTA image;
using the first OCTA image as a target result, and initializing convolution kernels and weights;
selecting a second quantity of images from the first quantity of images as a second image set, wherein the second quantity is less than the first quantity;
computing images in the second image set using the OCTA image computing method according to the initialized convolution kernels and weights to obtain a second OCTA image;
computing a residual between the second OCTA image and the target result;
accumulating the residual to obtain a loss function; and
optimizing the model according to the loss function until a preset condition is satisfied to obtain an optimized model, wherein the optimized model comprises optimized convolution kernels and weights corresponding to the optimized convolution kernels.

13. The method according to claim 12, wherein the step of computing the first quantity of images according to a preset algorithm to obtain a first OCTA image comprises:
computing the output OCTA image according to a following formula: $Angio = \frac{1}{M - 1} \sum \frac{\left|\left|{C}_{i}\right|-\left|{C}_{i + 1}\right|\right|}{\left(\left|{C}_{i}\right|+\left|{C}_{i + 1}\right|+Sigma\right)} ,$
wherein Angio is the output OCTA image, Cᵢ is the complex amplitude image corresponding to i-th image, Sigma is zero or a positive number, and Sigma is determined according to system noise, and *M* is the first quantity; and
performing low-pass filtering and denoising processing on the output OCTA image to obtain the first OCTA image.

14. The method according to claim 12 or 13, wherein the step of initializing the convolution kernels and the weights comprises:
assuming the number of convolution kernels is n, setting a center element of a first convolution kernel to 1 and remaining elements to 0; setting all elements at a distance of 1 from a center of a second convolution kernel to 1/L, where L is the number of all non-zero elements, and remaining elements to 0; and so on; and
initializing the weights to ${W}_{i} = \frac{1}{n}$, wherein Wᵢ is the weight corresponding to the i-th convolution kernel.

15. The method according to any one of claims 1-14, wherein before the step of performing Fourier transform on the OCT image, the method further comprises:
performing dispersion correction processing on the OCT image to correct dispersion of the image.

16. The method according to any one of claims 1-15, wherein after the step of performing Fourier transform on the OCT image to obtain a complex amplitude image, the method further comprises:
for each of the complex amplitude images, computing an absolute value of a signal corresponding to the complex amplitude image and using the absolute value as a real amplitude image;
for each of the real amplitude images, computing a logarithm of a signal corresponding to the real amplitude image to obtain a first structural image;
selecting one of the first structural images as a reference image, and determining first structural images other than the reference image as images to be corrected;
for each of the images to be corrected, computing a misalignment amount between the reference image and the image to be corrected; and
correcting the complex amplitude images corresponding to the images to be corrected based on all of the misalignment amounts to obtain corrected complex amplitude images.

17. An OCTA image computing apparatus, **characterized in that** the apparatus comprises:
an image acquisition module, configured to acquire a scanned image set, wherein the scanned image set comprises at least two OCT images obtained by scanning a target position;
an image transform module, configured to, for each of the OCT images, perform Fourier transform on the OCT image to obtain a complex amplitude image;
a convolution calculation module, configured to, based on at least two preset convolution kernels, perform convolution calculation on each of the complex amplitude images with each of the convolution kernels to obtain a sub-image set corresponding to each of the convolution kernels;
a determination module, configured to determine a first image set based on all of the sub-image sets; and
an OCTA image computing module, configured to compute images in the first image set to obtain an OCTA image.

18. An electronic device, **characterized by** comprising: a memory and a processor, wherein the memory stores a computer program, and the processor is configured to execute the computer program to implement the method according to any one of claims 1-16.

19. A computer-readable storage medium, **characterized in that** a computer program is stored on the computer-readable storage medium, wherein the computer program, when executed by a processor, implements the method according to any one of claims 1-16.

20. A computer program product, **characterized by** comprising a computer program, wherein the computer program, when executed by a processor, implements the method according to any one of claims 1-16.
